# EUROPÄISCHE PATENTSCHRIFT

(11) **EP 0 893 975 B1**
(45) Veröffentlichungstag und Bekanntmachung des Hinweises auf die Patenterteilung: **16.04.2003**
(21) Anmeldenummer: 96943906.6
(22) Anmeldetag: 10.12.1996
(51) Int. Cl.: A61C 8/00, A61C 13/20, A61F 2/02

(54) **MEDIZINISCHES IMPLANTAT**
MEDICAL IMPLANT
IMPLANT MEDICAL

(30) Priorität: 18.12.1995 CH 356595
(43) Veröffentlichungstag der Anmeldung: 03.02.1999
(73) Patentinhaber: Schug, Jens, 8458 Dorf (CH); Suhonen, Jouko, Horley, Surrey RH6 8QG (GB)
(72) Erfinder: Schug, Jens, 8458 Dorf (CH); Suhonen, Jouko, Horley, Surrey RH6 8QG (GB)
(74) Vertreter: Schreiber, Wolfgang
(86) Internationale Anmeldenummer: EP9605506
(87) Internationale Veröffentlichungsnummer: WO97022308

(56) Entgegenhaltungen:
- DE-A- 2 658 716
- DE-A- 4 432 831
- US-A- 4 671 768

## Beschreibung

Die vorliegende Erfindung betrifft ein medizinisches Implantat, das zum Einsetzen in einen Raum mit einem vorgegebenen Ausmass zum Ausfüllen desselben bestimmt ist, und ein Verfahren zur Herstellung dieses medizinischen tmplantates.

Es ist bekannt, dass Knochen bei Vorliegen einer Extraktionswunde sich derart verändem, dass der durch die Extraktionswunde entstandene Raum nach einer gewissen Zeit nicht mehr durch ein Implantat gefüllt werden kann, dessen Ausmass und Form genau dem extrahierten Hartgewebe entspricht.

Als Beispiel kann ein Zahnverlust genannt werden, welcher durch ein Trauma entsteht, wenn der Zahn ungünstig frakturiert und die Erhaltung der Wurzelfragmente nicht möglich ist. Somit muss man den frakturierten Zahn bzw. dessen Wurzelfragmente extrahieren. Als Ersatz verwendete man seit langem eine Brücke oder eine abnehmbare Prothese. Diese sind nachteilig, da man zu ihrer Verankerung Nachbarzähne einbeziehen und gegebenenfalls bearbeiten muss. Zur Behebung dieses Nachteils verwendet man gelegentlich Sofortimplantate an der Stelle des verlorenen Zahnes. Da diese bisher nur aus vorfabrizierten, genormten alloplastischen Materialien bestehen, kann die Extraktionsalveole nicht mit dem unbedingt erforderlichen exakt angepassten Sofortimplantat ausgefüllt werden. Dadurch entstehen zwangsläufig Hohlräume zwischen dem Rand der Alveole und dem Implantat. Diese Hohlräume füllen sich beim Heilungsvorgang durch schnell wachsendes Bindegewebe, welches eine vollständige Osseointegration des vorfabrizierten, genormten Implantates verhindert. Daher geht ein hoher Anteil der bisher verwendeten Sofortimplantate wieder verloren.

Die Folge eines Zahnverlustes ist bekanntlich Knochenatrophie im Bereich der Extraktionsalveole. Jeder Knochenschwund im Kieferbereich ist für den nachfolgenden Ersatz des verlorenen Zahnes äusserst ungünstig. Durch den Verlust von Knochenvolumen ist eine spätere Implantation einer künstlichen Zahnwurzel, also eines enossalen Implantates, schwierig. Da selbst bei einem normal ablaufenden Heilungsprozess einer Extraktionswunde Substanz des Kieferknochens im Bereich des Kieferkammes verlorengeht, ist es unmöglich, das implantat in der Position zu setzen, die derjenigen des extrahierten Zahnes bzw. dessen Wurzel exakt entspricht. Ein solches Implantat ist gegenüber der ursprünglichen, natürlichen Position in horizontaler und auch in vertikaler Ebene stark verschoben. Diese Tatsache wirkt sich für die Ästhetik und Funktion ungünstig aus.

Implantate sind auch zum Ersatz anderer Teile des Skeletts verwendbar. Ist beispielsweise der Unterkiefer von einem Tumor befallen, wird der vom Tumor befallene Bereich vom Kieferknochen abgetrennt und durch ein Implantat ersetzt. Auch hier treten die oben angeführten Schwierigkeiten auf.

Der vorliegenden Erfindung liegt die Aufgabe zugrunde, den Zeitraum zwischen erfolgtem, therapiebedingtem Hartgewebeverlust, z.B. Verlust der Knochensubstanz, insbesondere nach erfolgter Zahnextraktion, und dem Einsatz des Implantates so zu verkürzen, dass kein Knochenschwund im nennenswerten Ausmass einsetzen kann.

Der vorliegenden Erfindung liegt insbesondere die Aufgabe zugrunde, den Hartgewebeverlust infolge von Knochenresektion oder insbesondere bei erforderlicher Zahnextraktion, und den Einsatz des genau angepassten individuellen Implantates im Sinne eines sogenannten "Custom-Made-System" an der vorgesehenen Stelle im Rahmen einer einzigen therapeutischen Massnahme durchzuführen.

Diese Aufgabe wird durch die vorliegende Erfindung gelöst, welche ein medizinisches Implantat zum Einsetzen in einen Raum mit vorgegebenem Ausmass, z.B. der Extraktionsalveole, sowie ein Verfahren zur Herstellung dieses Implantates, betrifft.

Die Erfindung betrifft bevorzugt Zahnimplantate bzw. Zahnwurzelimplantate. Das Implantat hat beispielsweise den Vorteil, dass bei zahnärztlichen Eingriffen das Herstellen und Implantieren des individuellen Implantates im Rahmen einer einzigen therapeutischen Massnahme durchgeführt werden kann. Damit lässt sich Zeit einsparen und ein traumatisierender Zweiteingriff vermeiden. Es ist keine zweite Anästhesie notwendig und keine weitere Belastung des Patienten mit einem emeuten "chirurgischen Eingriff". Diese Imptantationsmassnahme können ausserhalb von Klinikzentren Zahnärzte mit normaler Berufsbefähigung im Rahmen ihrer Privatpraxis durchführen. Es wird eine hohe Akzeptanz durch den Patienten erreichbar sein, da der Gewebeverlust sofort und mit geringem Risiko ersetzt werden kann. So kann durch das sofortige Herstellen und Einsetzen des individuellen Implantates der Alveolarknochenschwund verhindert werden. Auch kann man andere Knochenfragmente mit einem passgenauen biokompatiblen, da biodegradierbaren, Passkörper ersetzen, der bei gewissen Ausführungen eine Fixtur aus alloplastischem Material enthält. Nachfolgend wird der Erfindungsgegenstand der Variante a) anhand der Zeichnungen beispielsweise näher erläutert:
**Figur 1:** Gusshöhlung in Form eines Abdruckes einer Zahnwurzel mit eingesetzten Formkernen;
Figur 2: Mit bioabbaubarem Material ausgegossene Gusshöhlung mit alternativer Anordnung von Formkernen;
Figur 3: Implantat mit noch nicht entfernten Formkemen;
**Figur 4:** Implantat nach Figur 1, das im Kieferknochen eingesetzt ist und Reservoire aufweist, welche anstelle der Formkeme entstanden sind und mit biologisch aktiven Substanzen gefüllt sind;
Figur 5: Teil des Unterkiefers zur Illustration einer weiteren Ausführungsform der Erfindung;
Figur 6: Formmasse, die mit einem bioabbaubaren Material zur Bildung eines dem Resektionsdefekt entsprechenden Implantates ausgegossen ist und Formkeme zur Reservoirbildung enthält;
Figur 7: Implantiertes Implantat mit gefüllten Reservoiren;
Figur 8: Unterkiefer mit darin fixiertem Implantat;
Figur 9: Schnitt durch einen Imptantat-Kem als Fixtur gemäss einer weiteren Ausführungsform der Erfindung;
Figur 9A: Ausschnitt aus der Figur 9 mit Variante einer strukturierten Innenseite des Implantatkernes nach Figur 9;
Figur 10: Aufsicht auf den Implantat-Kem nach Figur 9;
Figur 11: Dichtungsscheibe;
Figur 12: Drahtförmiger Formkern;
Figur 13: Formkern mit glattwandigem Schaft und Kopf mit Schraubgewinde;
Figur 14: Implantat-Kem nach Figur 9 mit eingesetzen Formkernen;
Figur 15: Implantat-Kem nach Figur 14 im Abdruck in der Formmasse eingesetzt;
Figur 16: Implantat nach Figur 15 mit gefüllten Reservoiren;
Figur 17: Deckschraube;
Figur 18: Eingesetztes Implantat mit durch eine Deckschraube verschlossenen Reservoiren;
Figur 19: Weitere Ausführungsform eines Implantat-Kernes;
Figur 20: Implantat-Kem nach Figur 9 mit teilweise durch bioabbaubares Material gefülltem Innenraum;
Figur 21: Implantat-Kem zum Einsetzen in eine Formmasse;
Figur 22: Mit bioabbaubarem Material ausgegossene Gusshöhlung mit Fixtur;
Figur 23: Fertiggestelltes Implantat;
Figur 24: Implantiertes Implantat;
Figur 25: Weitere Ausführungsform eines Implantat-Kernes mit Formkernen;
Figur 26: Implantat-Kern nach Figur 25 eingesetzt in eine Formmasse; und
Figur 27: Implantat mit einem Implantat-Kem nach Figur 25.

Ebenfalls Gegenstand der Erfindung ist das Verfahren zur Herstellung des medizinischen Implantates.

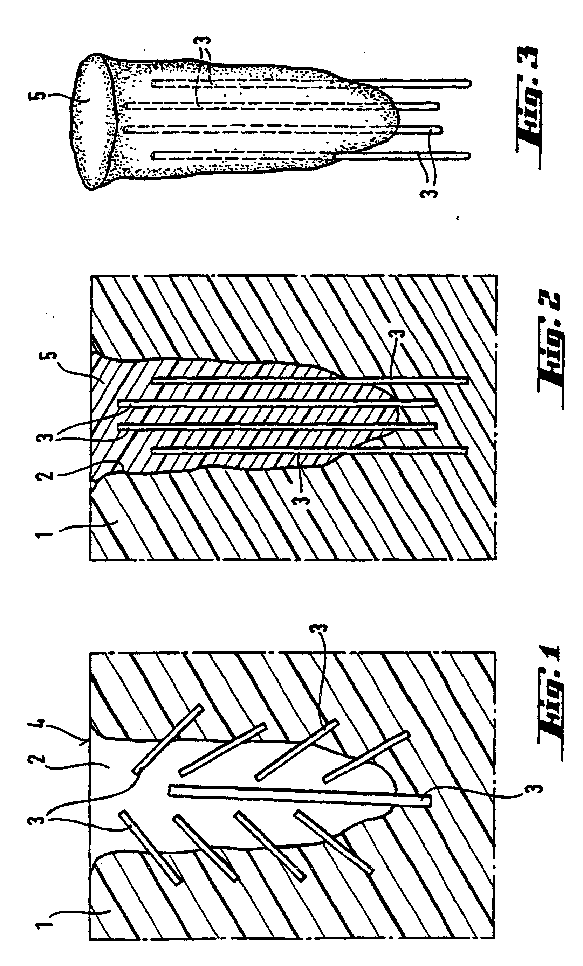

In den Figuren 1-4 ist eine erste Ausführungsform der Erfindung sowie ihr Herstellungsverfahren illustriert, wobei das Implantat einen durch Zahnextraktion entstandenen Knochendefekt auffüllen soll.

Fig.1: Zunächst wird der betroffene Zahn sorgfältig extrahiert. Falls eine Fraktur vorliegt, werden seine Fragmente, insbesondere der Wurzel, zusammengeklebt. Die kontaminierte Wurzeloberfläche des Zahnes wird aseptisch, mechanisch und/oder chemisch gereinigt. Der Zahn wird danach in einer an sich bekannten Weise bis zum Zahnhals in eine Formmasse 1 eingedrückt, welche ein steriles, elastisches Abformmaterial einer bekannten Art ist, beispielsweise Alginate, additions- und kondensationsvemetzte Silicone, Polyether, etc.. Nach dem Abbinden der Formmasse 1 wird der Zahn herausgezogen, womit in der Formmasse 1 eine Gusshöhlung 2 in Form eines Abdruckes des herzustellenden dentalen Implantates gebildet ist.

In dieser Formmasse werden nun von der Gusshöhlung her mehrere Formkerne 3 in Form von Drähten, bzw. Stäbchen aus glattflächigem, bruchfesten Material eingesetzt. Der Begriff Formkerne umfasst Gegenstände und Vorrichtungen, welche als Platzhalter für auffüllbare Reservoire bzw. Hohlräume verwendbar sind. Die draht- bzw. stäbchenförmigen Formkeme 3 werden dabei derart plaziert, dass sie das Niveau 4 des Limbus alveolaris nicht erreichen. Damit ist in der Formmasse 1 eine Gusshöhlung 2 ausgebildet, in welcher die Formkeme 3 so angeordnet sind wie in der Figur 1 dargestellt.

Diese Gusshöhlung 2 wird nun mit einem sich abbindenden plastischen Material mit mineralischen Bestandteilen auf der Basis von Calcium und Phosphat oder einem bei der Knochenneubildung bioabbaubaren Material oder Kombinationen ausgegossen.

Plastische Materialien mit mineralischen Bestandteilen auf der Basis von Calcium und Phosphat (CPHC: Calcium Phosphate Hydraulic Ciments) sind durch Kombinieren von teilweise neutralisierten Phosphatmineralien und Calciumsalzen nach dem in der Europäischen Patentanmeldung 416 761 beschriebenen Verfahren erhältlich. Geeignete Phosphatmineralien sind beispielsweise Ca(H₂PO₄)₂.H₂O [MCPM], CaHPO₄.H₂O [DCPD: Brushit], Ca₉(HPO₄)(PO₄)₅(OH) [CDHA: Calcium-Deficient Hydroxyapatite], Ca₃(PO₄)₂ [α-oder β-Tricalciumphosphat: TCP], Ca₅(PO₄)₃(OH) [OHAP: Hydroxyapatit], Ca₂₈(PO₄)₁₅(CO₃)₃(OH)₅ [CHAP: Carbonated Hydroxyapatite], Ca₂P₂O₇ [CPP: Calcium pyrophosphat], CaSO₄.0,5H₂O [CSH: "Plaster of Paris"], CaSO₄.2H₂O [CSD: Gips], CaCO₃ [CC: Calcit] oder Mischungen davon.

Bevorzugt ist ein plastisches Material mit mineralischen Bestandteilen auf der Basis von Calcium und Phosphat mit folgenden Mengenanteilen: 60% - 80% β-TCP, 40% - 20% MCPM, wässriger Lösung enthaltend P₂O₇⁴⁻ und SO₄²⁻ Ionen, sowie Additiven vom Typ Celluloseether, z.B. HPMC (0,5% -1,0 %) oder Polysaccharide.

Für die Knochenneubildung geeignete Materialien haben osteokonduktive Eigenschaften und sind vorzugsweise biodegradierbar und biokompatibel. Osteokonduktive Materialien steuern bei der Knochenreparatur "Guided Bone Repair" (GBR) den Aufbau einer Struktur für die Knochenneubildung.

Biodegradierbare und biokompatible Materialien sind allgemein bekannt, z.B. aliphatische Polyester vom Typ polymere Polyglycolsäuren (PGA = Polyglycolic Acid) oder Polylactat (PLA = Polylactic Acid) oder auch deren Copolymere (PGA/PLA), enantiomere Formen und racemische Mischungen in unterschiedlichen Verhältnisanteilen, z.B. Poly-L-Lactat (PLLA), Poly-D-Lactat (PDLA), Poly-DL-Lactat (PDLLA), L-Lactat/DL-Lactat, oder L-Lactal/D-Lactat. Diese Materialien sind nicht nur bioabbaubar, sondern auch biokompatibel. PGA und PLA haben Metabolismuswege im menschlichen Körper. Weiter sind PGA und PLA-Materialien nicht immunogen, das heisst, in Säugetieren sind keine Immunreaktionen auf diese Materialien hervorgerufen worden. Geeignet sind beispielsweise Handelsprodukte vom Typ Biofix®, welche bei der Fa. Bioscience, SF-33721 Tampere, kommerziell erhältlich sind.

Geeignete aliphatische Polyester mit osteokonduktiven Eigenschaften sind femer PLA-Copolymere, z.B. Lactat/Tetramethylenglycolid Copolymere, Lactat/Trimethylencarbonat Copolymere, Lactat/α-Valerolacton Copolymere, Lactat/ε-Caprolacton Copolymere, Polydepsipeptide (Glycin-DL-Lactatcopolymer oder PLA/Ethylenoxid Copolymere (PLA/PEO), Polylactid-polyglycolid-Copolymere oder Polylactid-Ethylenoxid- Copolymere, oder Polyhydroxyalkanoate, z.B. PHB [Poly(β-hydroxybutyrat)], PHB/PHA (Polyhydroxybutyrat/-valerat), PCL [Poly(ε-caprolacton)], PDS [Poly (p-dioxanon)], Polyanhydride, Polyäpfelsäure (β) oder Polyäpfelsäureester.

Für die Knochenneubildung geeignete Materialien mit osteokonduktiven Eigenschaften sind femer Vinylpolymere, z.B. auf der Basis Polyvinylalkohol (PVA), Poly-β-maleinsäure, aliphatische Polyamide, aliphatische Polyurethane, z.B. Polyurethane aus Polyethylenglycol-(PEG)-dioien oder Polycaprolacton-diolen und Diisocyanaten, wie 1,4-Methylendiisocyanat, Polyorthoester, z. B.vom Typ Alzamer® (Alza Corp.) oder DETOSU, aliphatische Polyanhydride, Polypeptide, z.b. synthetische Polyaminosäuren und Poly-α-aminosäuren, z.B. Poly-β-lysin oder Polybenzylglutamat, Polyphosphate, Polysaccharide, z.B. Dextranderivate, Chitin- und Chitosanderivate oder Hyaluronsäureester, modifizierte Proteine, z.B. vemetztes Collagen oder Fibrin, oder modifizierte Kohlehydratpolymere.

Geeignet sind ferner auch deren Gemische, "Composites", sowie die Block- und Pfropf-Copolyrnere der genannten Polymere und Copolymere.

Die genannten plastischen Materialien können durch geeignete Füllinstrumente, z.B.Injektionsinstrumenten, in die Gusshöhlung 2 eingebracht werden.

Fig. 2: Figur 2 zeigt eine mit bioabbaubarem Material ausgegossene Gusshöhlung, welche nach dem Abbinden des plastischen Materials die Form des Implantates 5 aufweist. Die Formkeme 3 weisen hier im Vergleich mit der Ausführung nach Figur 1 eine alternative Anordnung und Ausbildung auf.

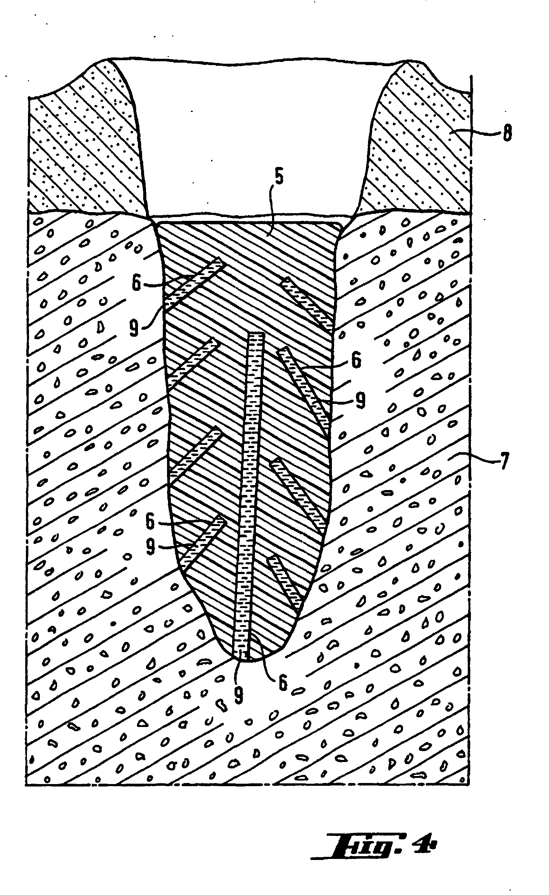

Fig. 3: Nach dem Abbinden des Materials zum Implantat 5 wird dieses zusammen mit den Formkemen 3 aus der Gusshöhlung 2 entnommen. Danach werden die drahtförmigen Formkeme 3 aus dem Implantat 5 herausgezogen. Damit werden im Implantat 5 kanal- oder kapillarförmige Räume oder Zwischenräume 6 gebildet, die als Reservoire für biologisch aktive Substanzen (sogenannte Aktivsubstanzen) dienen.

Geeignete biologisch aktive Substanzen werden in die Reservoire eingefüllt. Sie haben osteoinduktive Eigenschaften und können das biologische Verhalten benachbarter Zellen beeinflussen, beispielsweise die Zellteilung oder Knochenbildung anregen, z.B. durch Bildung von mesenchymalen Zellen, Endothelgewebe, Pericyten, Osteoclasten, Osteoblasten usw.. Geeignete biologisch aktive Substanzen mit osteoinduktiven Eigenschaften sind z.B. Hormone, Proteine oder Wachstumsfaktoren auf Protein- oder Lipidbasis, welche unter Begriffen wie *Epidermal Growth Factor* (EGF), *Vaseular Epideimal Growth Factor* (VEGF), *Fibroblast Growth Factor* (FGF), *Platelet Derived Growth Factor* (PDGF), *Transforming Growth Factor-β* (TGF-β), z.B. vom Typ TGF-β-1, -2 oder -3, *Insulin-like Growth Factor* (IGF-I) und IGF-II), *Nerve Growth Factor* (NGF), *Bone Morphogenic Proteins* (BMP), z.B. BMP-3 (*Osteogenin*), -2 (BMP 2A), -4 (BMP 2B), -5,-6,-7 (*Osteogenic Protein-1),* sowie Proteine, welche unter Begriffen wie *Parathyroid Hormone* (PTH), z.B. PTH-Fragmente, z.B. PTH 1-34 und Derivate davon, *Parathyroid Hormone Related Protein* (PTHrP), z.B. PTHrP-Fragmente, z.B. PTHrP 1-34 und Derivate davon Osteoglycin, *Cartilage Induction factor und Skeletal Growth Factor* bekannt sind. Knochenwachstumsfaktoren auf Lipidbasis umfassen Prostanoide, welche unter Begriffen, wie Prostaglandin A, D, E, F, I und Derivate davon sowie Prostazyklin bekannt sind.

Proteine (Wirkstoffkomponente) mit den Eigenschaften eines transformierenden Wachstumsfaktors vom Typ Beta (TGF-β) sind bekannt und in dem Übersichtsartikel von *A. B. Roberts und M.B. Sporn, The transforming growth factor -βs, im Handbook of Experimental Pharmacology. Peptide Growth Factors and Their Receptors, M. B. Sporn und A.B. Roberts Herausgeber, Springer Verlag Berlin, New York, Seiten 419-472*, beschrieben.

Proteine vom Typ TGF-β humanen Ursprungs sind bekannt und in dem Übersichtsartikel von *D.A. Cox, Transforming Growth Factor-Beta 3, Cell Biology International*, *19(5): 357-371 (1995)* beschrieben.

Rekombinante Proteine vom Typ TGF-β sind bekannt und in folgenden Übersichtsartikeln beschrieben: *Lionel Bourdrel et al., Recombinant Human Transforming Growth Factor-β1: Expression by Chinese Hamster Ovary Cells. Isolation and Chracterization, Protein Expression and Purification 4: 130-140 (1993); M.P. Schlunegger and M.G.Grütter, An unusual feature revealed by the crystal structure at a resolution of human transforming growth factor-*β *2, Nature 358: 430-434 (1992); S.Runser and N.Cerletti, Transforming Growth Factors β: conformational stability and features of the denaturation of recombinant human transforming growth factors-β 2 and β 3*, *Biotechnol. Appl. Biochem. 22: 39-53 (1995)*.

Proteine mit den Eigenschaften eines transformierenden Wachstumsfaktors vom Typ Beta (TGF-β) ausgewählt aus der Gruppe bestehend aus TGF-β 1, TGF-β 2, TGF-β 3, TGF-β 5 und Knochen-morphogenen Proteinen (BMP: bone morphogenic proteins) sind bekannt und in dem Übersichtsartikel.von *D.M. Kingsley, The TGF-β superfamily: new members, new receptors, and new gentic tests of function in different organisms*, *Genes and Development 8:133-146(1994)* beschrieben.

Weitere Substanzen, die in die genannten Reservoirs eingefüllt werden können, sind Aktivsubstanzen, welche die Knochenresorption hemmen, z.B. Bisphosphonate vom Typ Aredia®, Nitrate, z.B. Nitroglycerin, Ipriflavon, Wirkstoffe, die an nukleare Rezeptoren binden, z.B. Östradiol, Enzymhemmer, welche Knochenmatrix-abbauende Enzyme blockieren, z.B. Kollagenase-Hemmstoffe, Stromelysin-Hemmstoffe, Cathepsin L, K-Hemmstoffe, Stoffe, welche die Osteoklastenfunktion hemmen, z.B. Carboanhydrase-Hemmstoffe oder Hemmstoffe der osteoklastischen Protonenpumpe usw..

Weitere Aktivsubstanzen sind solche, die eine Wirksamkeit gegen Implantopathogene (Parodontopathogene) besitzen, z.B. Antibiotika, Antikörper (mono-, polyklonale), Entzündungshemmstoffe, Prostaglandinhemmer, Wirkstoffe mit immunsuppressiver Wirkung, z.B. (bio-)synthetische Immunsuppressiva, Wirkstoffe mit revaskularisationsfördemder Wirkung, z.B. gefässbildende Substanzen, durchblutungsfördernde Wirkstoffe, oder Analgetika. Vor dem Einsetzen des Implantates füllt man den zu verabreichenden biologisch aktiven Stoff oder Kombinationen solcher Stoffe in die Reservoire 6 ein. Die genannten biologisch aktiven Stoffe können beispielsweise mittels herkömmlicher ärztlicher Injektionsinstrumente in die Reservoire eingefüllt werden.

Das mit biologisch aktiver Substanz gefüllte Implantat stellt ein Abgabesystem ("dispensing unit") dar, welches eine Dosis der abzugebenden Substanz enthält und diese in einem festgelegten Zeitraum freisetzt. Ein Abgabesystem im Sinne dieser Definition enthält eine Dosiseinheit der zu verabreichenden Substanz oder Bruchteile oder Vielfache davon. Die Abgabe kann spontan, z.B. durch Diffusion oder Erosion des Systems durch Einwirken von Körperflüssigkeit, erfolgen.

Fig. 4: Man setzt das passgenaue Implantat 5 in die Alveole im Kieferknochen 7 ein, wobei in der Figur 4 zusätzlich die Gingiva 8 angedeutet ist. Dabei werden die Substanzen 9 aus dem implantiertem Implantat freigesetzt.

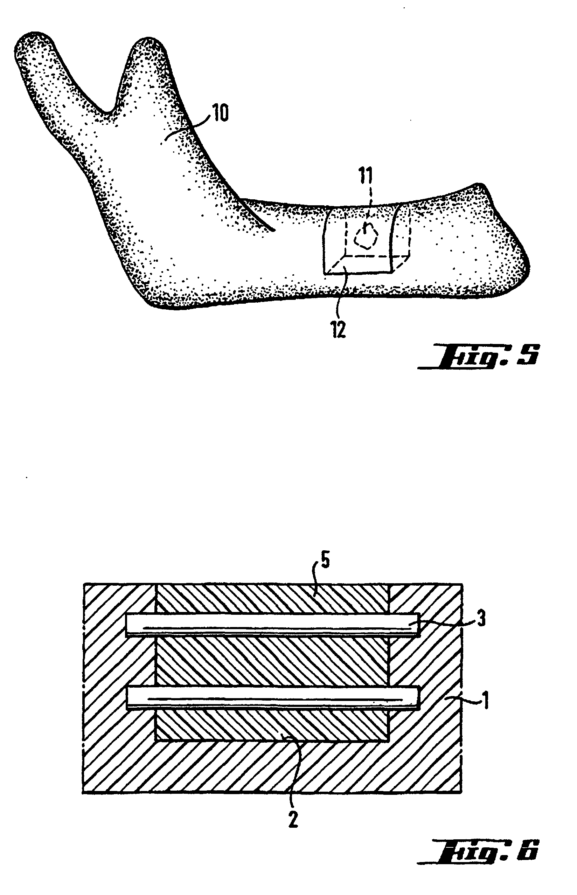

Eine andere Ausführungsform der Erfindung, welche sich insbesondere zur Verwendung als Abgabesystem eignet, ist in den Figuren 5 - 9 dargestellt:
Fig.5: Figur 5 zeigt einen Unterkiefer 10, In welchem sich ein Tumor 11 gebildet hat. Der mit dem Tumor 11 befallene Knochenteil 12 wird aus dem Unterkiefer 10 entfernt und danach wird in der Formmasse 1, siehe die Schnittansicht nach Figur 6, ein Abdruck geformt. In der Gusshöhlung 2 werden die Formkeme 3 so angeordnet, dass sie mit ihren Enden in die Formmasse 1 hineinragen. Die in der Figur 6 gezeigte Anordnung der Formkerne 3 ist beispielhaft wiedergegeben und kann variieren. Nach dem Einbringen der Formkerne 3 wird die Gusshöhlung 2 mit einem bioabbaubaren Material ausgegossen, das sich zum Implantat 5 abbindet.

Fig. 6: Der gemäss Darstellung in der Figur 6 aus dem Unterkiefer 10 entfernte Knochenteil 12 wird gegenüber der Darstellung in Figur 5 um 180° gedreht in die Formmasse 1 hineingedrückt, so dass sich der gemäss Darstellung in Figur 6 obere, gerundete Abschnitt des Knochenteils 12 in der Formmasse 1 gemäss Figur 6 unten befindet. Die im Implantat 5 nach dem Entfernen der Formkerne 3 verbleibenden Räume oder Reservoire 6 werden, wie oben beschrieben, mit einer oder mehreren Aktivsubstanzen 9 gefüllt.

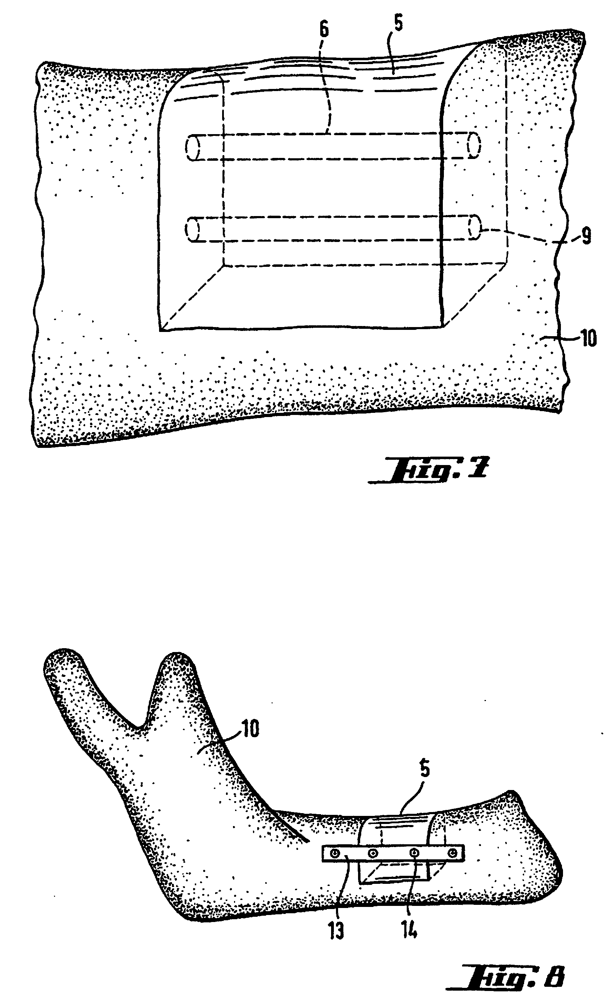

Fig. 7: Das Implantat 5 wird, wie in der Figur 7 dargestellt, an der vorgesehenen Stelle im Unterkiefer 10 implantiert.

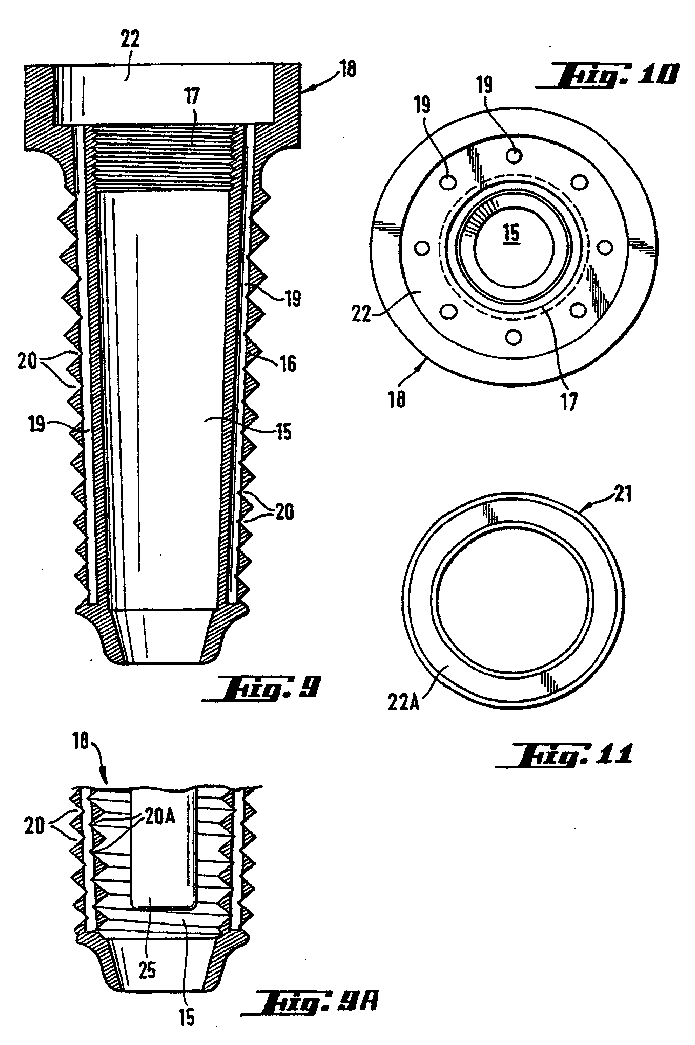

Fig. 8: Im Bedarfsfall kann man das Implantat 5 mittels einer Platte 13 im Kieferknochen fixieren, wobei die Platte 13 und auch die dazugehörigen Schrauben 14 ebenfalls aus einem bioabbaubaren Material bestehen können.

### Anhand der Figuren 9-18 wird eine weitere Ausführungsform der Erfindung beschrieben:

Fig. 9: Figur 9 zeigt eine Schnittansicht eines Implantat-Kemes, der gleichzeitig als Fixtur dient. Der Begriff Fixtur bezeichnet den enossalen Teil (innerhalb des Kieferknochens) eines Implantates, welcher den aus dem Kieferknochen herausragenden Teil eines Implantataufbaus (sichtbarer Implantatanteil) aufnimmt. Ein solcher Implantat-Kern besteht aus einem alloplastischen, osseointegrierbaren Material, z.B. Titan, Frialit, etc.. Er weist die Form eines langgestreckten Hohlkörpers 18 mit einem Innenraum 15 auf. Seine Aussenseite ist strukturiert, beispielsweise in Form eines Schraubgewindes 16. Andere Ausbildungen sehen auch eine Strukturierung der Innenseite vor. Beim oberen Ende des Innenraumes 15 ist ein Innengewinde 17 ausgebildet. In der Wand des Hohlkörpers 18 sind parallel zum Innenraum 15 verlaufende Hohlräume 19 vorhanden und sind oben offen und unten, d.h. beim vom Innengewinde 17 entfernten Ende, verschlossen. Eine andere Anordnung der Hohlräume 19 altemativ zur parallelen Anordnung zum Innenraum 15 ist möglich. Aus der Figur 9 ist ersichtlich, dass sich der Hohlkörper 18 zum unteren Ende, d.h. zur Wurzelspitze hin, verjüngt. Das Innengewinde 17 beim oberen, breiteren Ende, d.h. im Bereich des Limbus alveolaris, dient nicht nur als Verschluss für die Reservoire von Aktivsubstanzen, wie weiter hinten noch erläutert wird, sondem auch für die spätere Befestigung der Suprastruktur bei der Wiederherstellung eines Zahnes oder zur Befestigung von Prothesen mit Hilfe des Implantates. Die Hohlräume 19 in der Wand des Hohlkörpers 18 sind derart angeordnet, dass sie die Wellentäler des Schraubgewindes 16, bzw. einer jeweiligen Struktur der Wand des Hohlkörpers 18 schneiden. Ausserdem sind in der Wand Perforationen 20 vorhanden, welche von den Hohlräumen 19 zur Aussenfläche des Hohlkörpers verlaufen.

Fig. 9A: Eine alternative Anordnung von Perforationen ist in der Fig. 9A dargestellt, welche einen Ausschnitt aus der Fig. 9 darstellt. Bei dieser Ausbildung ist auch die Innenfläche des Hohlkörpers 18 strukturiert Dabei sind weitere Perforationen 20A vorhanden, die von den Hohlräumen 19 zur Innenfläche des Hohlkörpers 18 verlaufen, also die Hohlräume 19 mit dem Innenraum 15 des Hohlkörpers 18 verbinden. Es ist auch eine Ausführung vorgesehen, bei welcher nur die Innenfläche des Hohlkörpers 18 entsprechend strukturiert ist.

Fig. 10: Figur 10 stellt die Aufsicht auf den Implantat-Kern nach Figur 9 dar.

Fig. 11: Figur 11 zeigt einen Dichtungsring 21, der zum Einsetzen in den oberen, vertieften Endabschnitt 22 des Hohlkörpers 18 bestimmt ist. Dieser Dichtungsring 21 kann aus Siliconkautschuk oder aus Weichgold bestehen oder einen plastisch abdichtenden Metallwulst aufweisen, so dass dieser bei eingesetztem Dichtungsring 21 im Bereich 22 A die Zutrittsöffnungen zu den Hohlräumen 19 und zum Hohlraum 15 sicher gegen äussere Einflüsse, z.B. Eindringen von Pathogenen (Bakterien, Pilze, Viren, etc.), abschliesst.

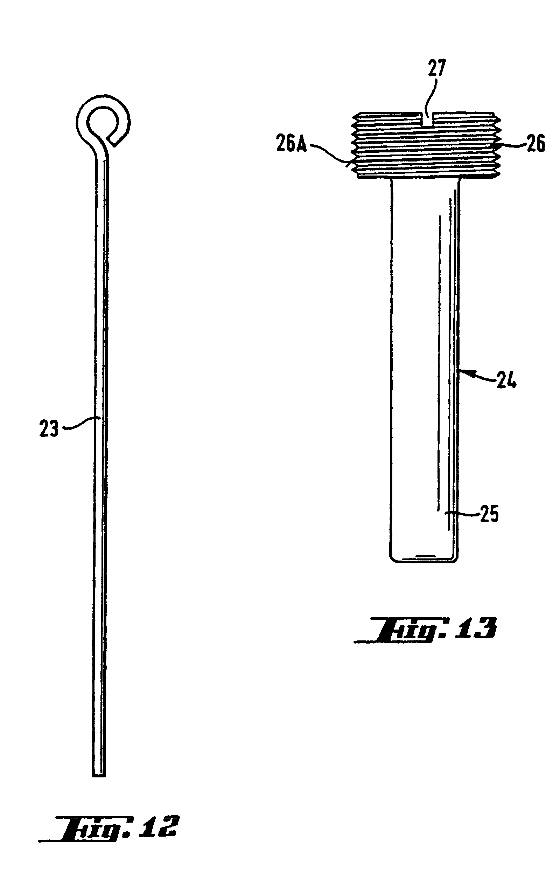

Fig. 12: Figur 12 zeigt einen drahtförmigen formkem 23. Sein Durchmesser ist derart bemessen, dass er in einen jeweiligen Hohlraum 19 eingeschoben werden kann.

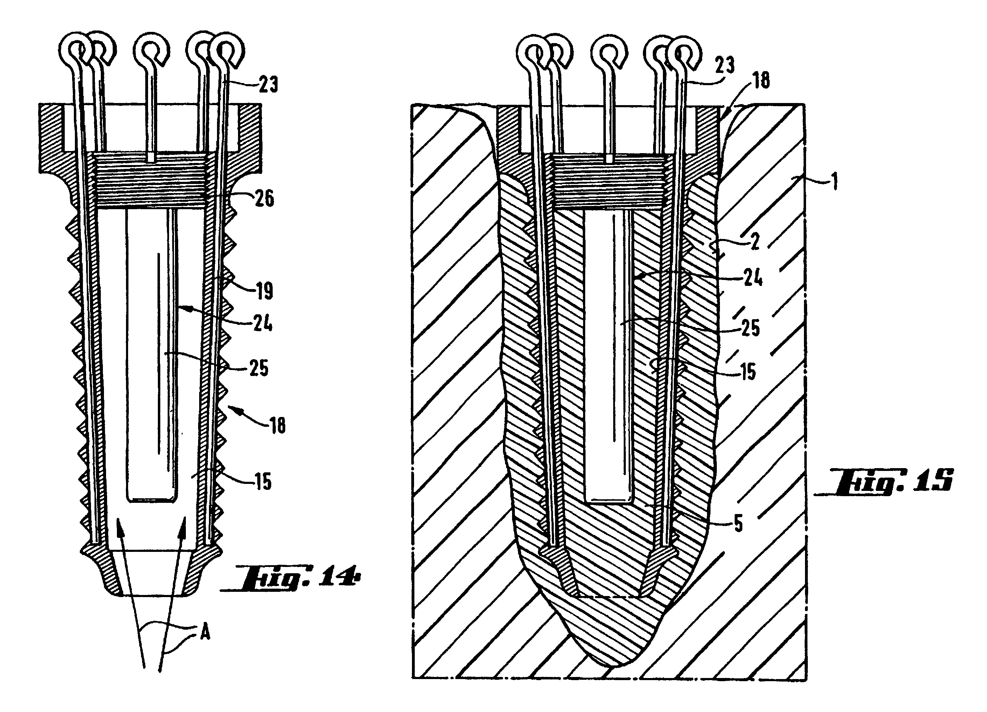

Fig. 13: In der Figur 13 ist ein weiterer Formkern 24 abgeblldet, der aus einem Schaft 25 und einem Kopf 26 besteht, der ein Aussengewinde 26 A sowie oben einen Schlitz 27 zur Aufnahme eines schraubendreherähnlichen Werkzeuges aufweist.

Fig. 14: Zur Herstellung eines Implantates wird vorerst der den Schaft 25 und Kopf 26 aufweisende Formkem 24 In den Innenraum 15 des Hohlkörpers 18 eingeschraubt. Weiter werden die drahtförmigen Formkeme 23 in die entsprechenden Hohlräume 19 hineingeschoben. In einem ersten Schritt wird nun von unten her in Richtung der Pfeile A eine erste Menge eines plastischen bioabbaubaren Materials in den Innenraum 15 eingebracht.

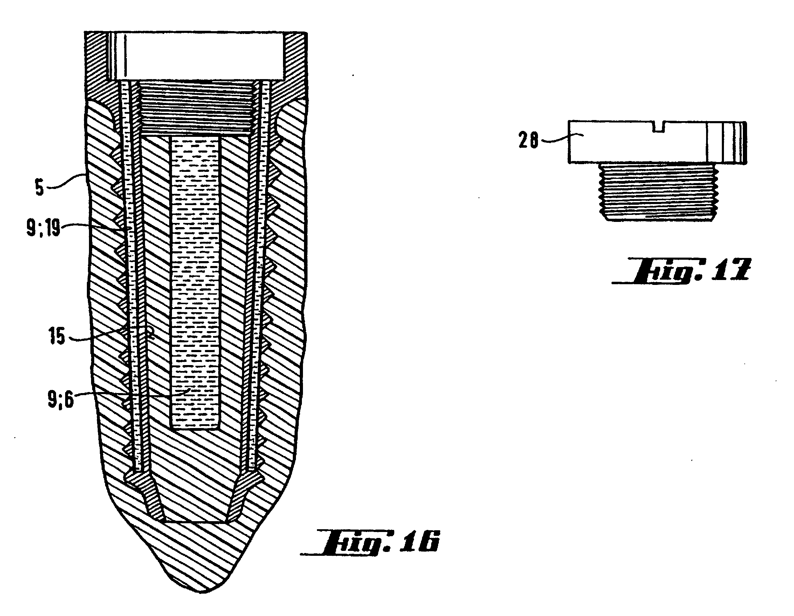

Fig. 15: In der Formmasse 1 ist ein Abdruck der durch das Implantat zu ersetzenden Zahnwurzel, also die Gusshöhlung 2, hergestellt worden. In die Gusshöhlung 2 wird in einem zweiten Schritt eine zweite Menge des bioabbaubaren Materials eingefüllt und in einem dritten Schritt der mit den Formkemen 23 und 24 ausgerüstete und bereits innen gefüllte Hohlkörper 18 in einem dritten Schritt in das noch fliessfähige bioabbaubare Material hineingedrückt. Zum Einfüllen wird das bioabbaubare Material je nach dessen Aufbau durch Wärme (in speziellen Applikationsspritzen oder auf anderem Weg) verflüssigt, oder es liegt in einer flüssigen oder plastischen Form bei Raumtemperatur vor. Die Erstarrung bzw. das Abbinden erfolgt entweder durch Abkühlung des thermoplastischen Materials oder durch eine chemische Reaktion (z.B. 2-Komponenten-Reaktion, fotochemisch oder Polymerisation etc.). Aus der Figur 15 ist ersichtlich, wie der Zwischenraum zwischen der Aussenwand des Hohlkörpers 18 und der Innenwand der Gusshöhlung 2 in der Formmasse 1 von diesem Material ausgefüllt wird. Nach dem Abbinden des Materials entsteht das Implantat 5, in welchem der Hohlkörper 18 eingebettet ist, der noch die Formkörper 23 und 24 trägt. Das Implantat 5 wird aus der Formmasse 1 entfernt und danach werden die drahtförmigen Formkerne 23 und der Formkem 24 aus dem Implantat 5 herausgezogen, bzw. herausgeschraubt.

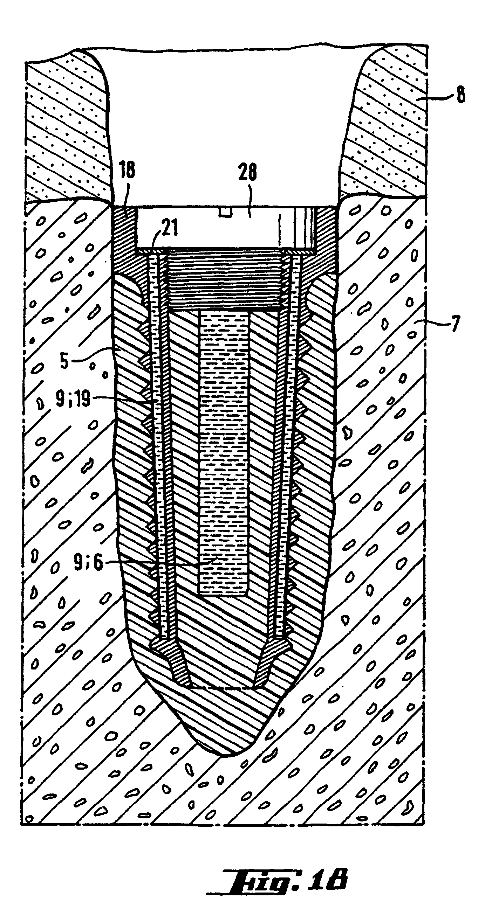

Fig. 16. Fig. 17: In die verbleibenden Räume 6 können die weiter vorn beschriebenen biologisch aktiven Substanzen eingefüllt werden. Die Räume 6 werden oben durch die Deckschraube 28 (Fig. 17) verschlossen. Damit ist ein Implantat 5 gebildet, das innen eine alloplastische Fixtur (Hohlkörper 18) aufweist, die von einem bioabbaubaren Material umgeben ist. Es sind Reservoire 19, 15 für Aktivsubstanzen vorhanden. Das der extrahierten Wurzel entsprechende, anatomisch replizierte Implantat ist zum Einsetzen in die Extraktionsalveole bereit.

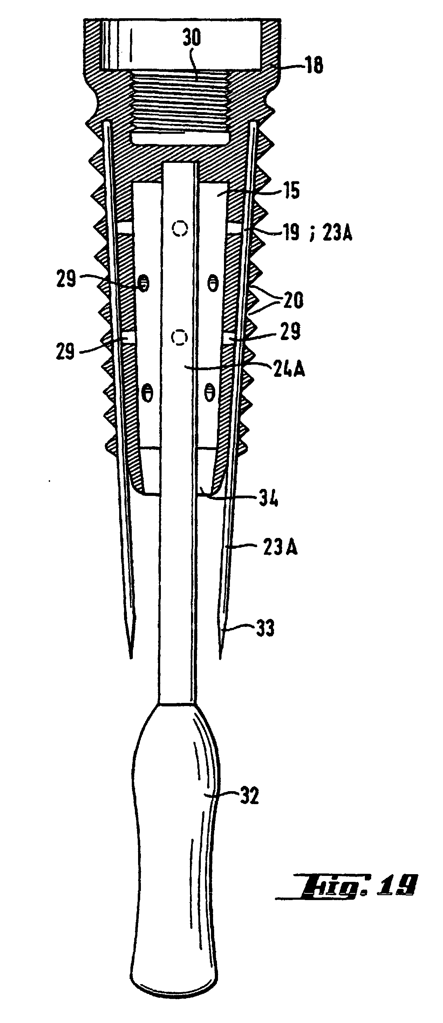

Fig. 18: Die Figur 18 zeigt das in den Kieferknochen 7 eingesetzte Implantat 5, wobei auch die Gingiva gezeichnet Ist. Zwischen der Deckschraube 28 und dem Hohlkörper 18 ist der Dichtungsring 21 angeordnet, der die Innenräume gegen äussere Einflüsse, z.B. gegen Bakterien, absperrt.

Das Implantat 5 passt exakt in die Extraktionsalveole, und es kann durch den engen Kontakt zum umgebenden Knochen eine Einheilung per primam erfolgen. Das bioabbaubare Material beginnt zu resorbieren. Werden durch den Biodegradationsprozess die Reservoire geöffnet, kommt es zur Freisetzung der Wachstumsfaktoren und/oder Aktivsubstanzen.

Dies führt zur beschleunigten und gesteuerten Knochenneubildung und einer qualitativ optimierten Osseointegration des alloplastischen Implantat-Kemes.

Eine weitere Ausführung wird nachfolgend unter Bezugnahme auf die Figuren 19-24 beschrieben:
Fig. 19: Der Hohlkörper 18, der aus einem ossointegrierbaren Material besteht, weist in der Mitte einen Innenraum 15 auf. Entlang der strukturierten Aussenwand verlaufen langgestreckte Hohlräume 19, entsprechend dem vorgängig beschriebenen Ausführungsbeispiel. Die Hohlräume 19 kreuzen die Wellentäler der strukturierten Aussenwand, so dass Perforationen 20 gebildet sind, die eine Verbindung zwischen den Hohlräumen 19 und den Aussenflächen des Hohlkörpers 18 bilden. Bei der Innenwand des Innenraumes 15 sind weitere Durchtritte oder Perforationen29 zu den Aussenffächen ausgebildet, wobei auch bei anderen Ausführungsformen solche Durchtritte 29 vorhanden sein können. Der später als Fixtur dienende Hohlkörper 18 weist oben ein Schraubgewinde 30 auf, in welchem eine temporäre Deckschraube 31 eingeschraubt werden kann, wobei das Schraubgewinde 30 zur späteren Fixierung des Zahnaufbaus dient

In den Innenraum 15 ist von unten her ein stangenförmiger Formkem 24 A mit Griff eingesteckt, der unten in einem Handgriff 32 endet. In den entlang der Aussenwand verlaufenden Hohlräumen 19 sind von unten her drahtförmige Formkerne 23 A eingesteckt, die beim unteren Ende eine Spitze 33 aufweisen.

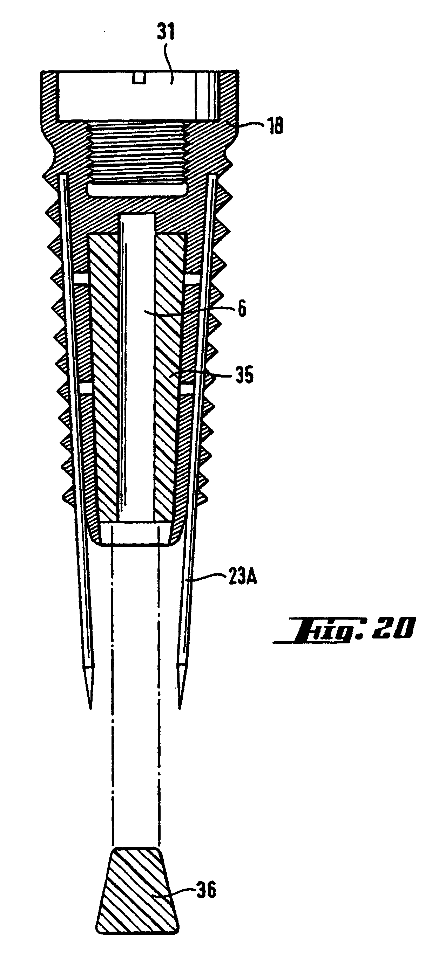

Fig. 20: Als erster Schritt wird von der unteren Oeffnung 34 her und durch die Durchtritte 29 bioabbaubares Material in den Ringraum zwischen dem Formkem 24 A und der Innenwand des Hohlkörpers 18 eingespritzt. Nach dem Abbinden dieses Materials wird der Formkem 24 A herausgezogen, so dass im Innenraum 15 eine Schicht 35 des bioabbaubaren Materials entsteht.

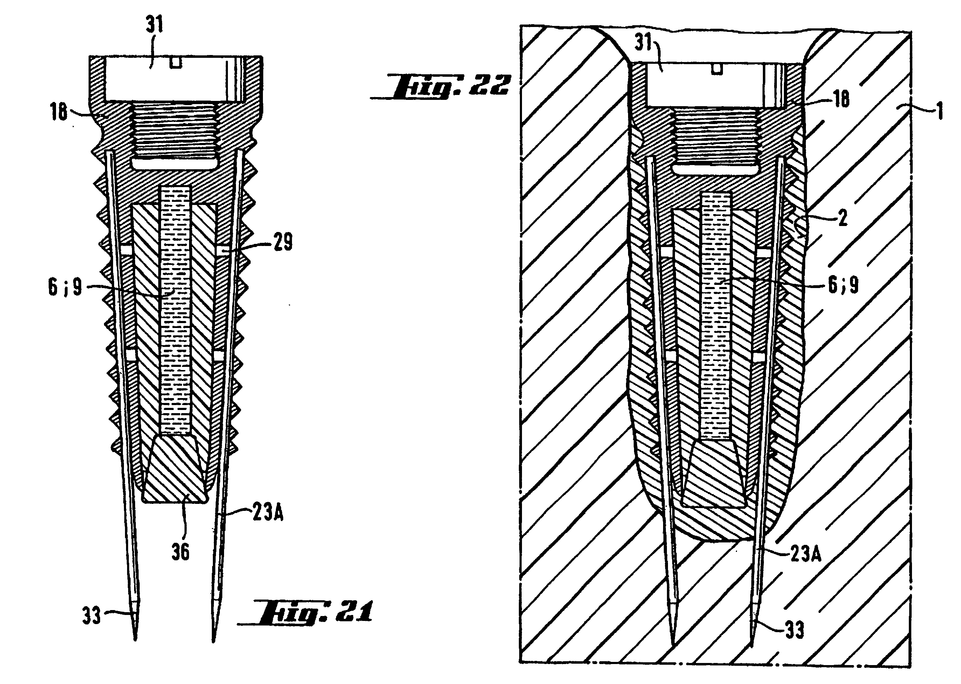

Fig. 21: In den Hohlraum 6 wird eine erste Menge der genannten Aktivsubstanzen 9 eingefüllt und der Hohlraum 6 unten mit einem zapfenförmigen Verschluss 36 verschlossen. Der Verschluss 36 besteht ebenfalls aus dem bioabbaubaren Material.

Fig. 22: Von der zu ersetzenden Zahnwurzel wird in der Formmasse 1 in bekannter Weise der Abdruck hergestellt, d.h. die Gusshöhlung 2 ausgebildet. Das bioabbaubare Material wird in die Gusshöhlung 2 eingefüllt und danach der Implantat-Kern gemäss der Figur 21 in die Gusshöhlung 2 eingesetzt, wobei die drahtförmigen Formkeme 23 mit ihren Spitzen 33 in die Formmasse 1 eindringen.

Fig. 23: Nach dem Abbinden bzw. Erstarren des eingefüllten bioabbaubaren Materials wird das nunmehr gebildete Implantat 5 aus der Gusshöhlung 2 entfernt und die drahtförmigen Formkerne 23 A unten aus dem Implantat-Kern herausgezogen und jeweilige medizinische Substanzen in die nun vorhandenen Räume 19 eingefüllt. Die unteren Oeffnungen 37 der gefüllten Räume 19 werden danach verschlossen. Dazu kann z.B. mittels einem rotierenden zahnärztlichen Werkzeug, beispielsweise einem Rosenbohrer bei den Bereichen der Oeffnungen 37 Reibungswärme erzeugt werden, so das bei diesen Stellen das bioabbaubare Material verschmolzen wird, so dass die Oeffnungen 37 verschlossen werden.

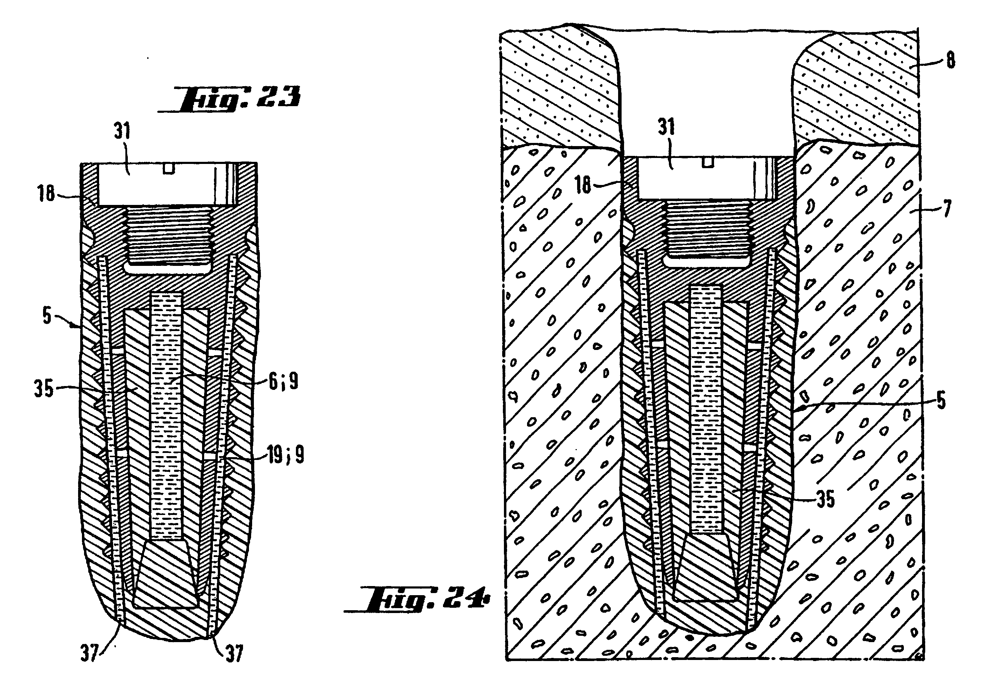

Fig. 24: Das nun fertiggestellte Implantat 5 kann gemäss der Darstellung nach Figur 24 in die Extraktionsalveole implantiert werden.

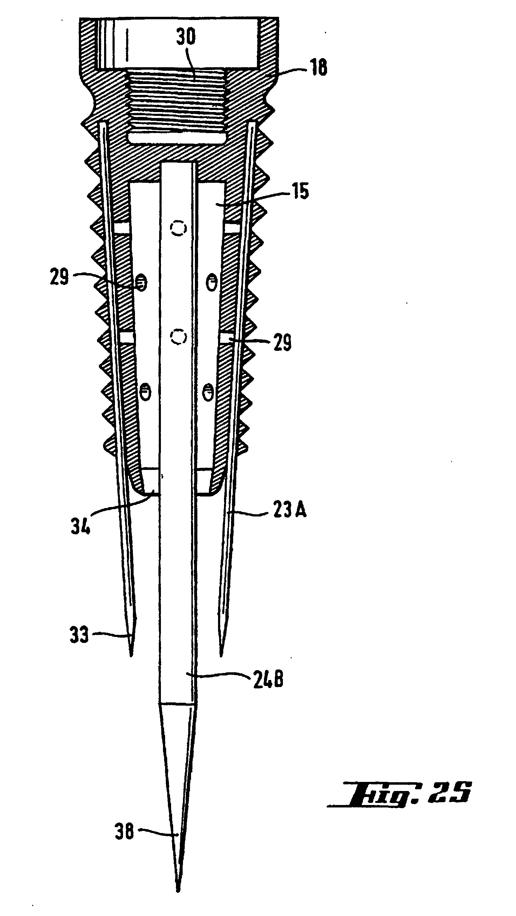

Anhand der Figuren 25-27 wird eine weitere Ausführungsform beschrieben. Die Verfahrensschritte sind den weiter vom beschriebenen Verfahrensschritten analog. Daher wird im folgenden das Verfahren vereinfacht beschrieben:
Fig. 25: Der Hohlkörper 18 bzw. Implantat-Kern ist analog zur Ausführungsform nach der Figur 19. Jedoch endet der stabförmige Formkern 24 unten nicht in einem Handgriff, sondern in der Spitze 38.

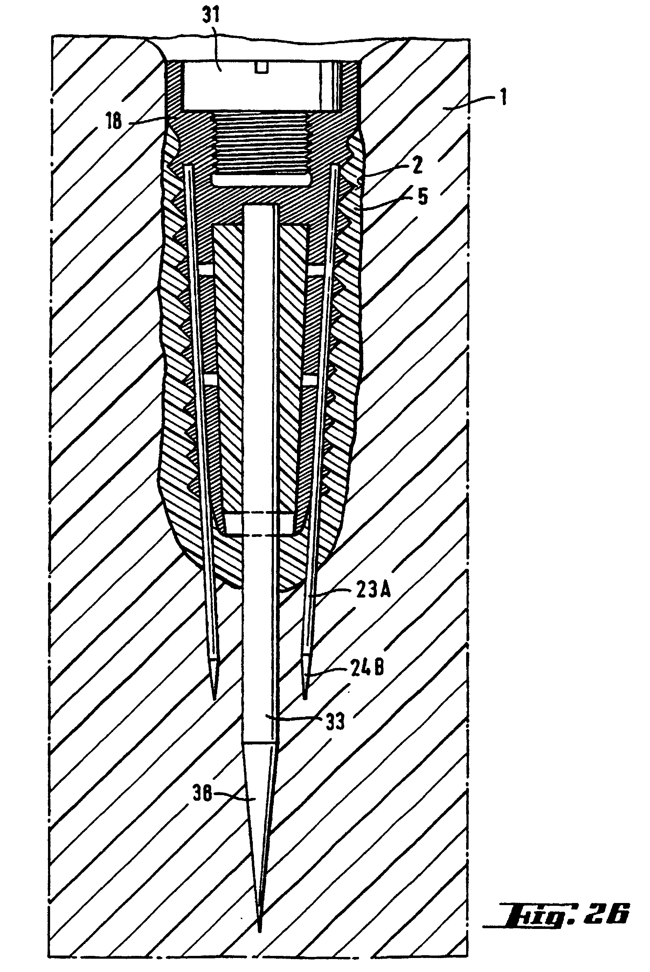

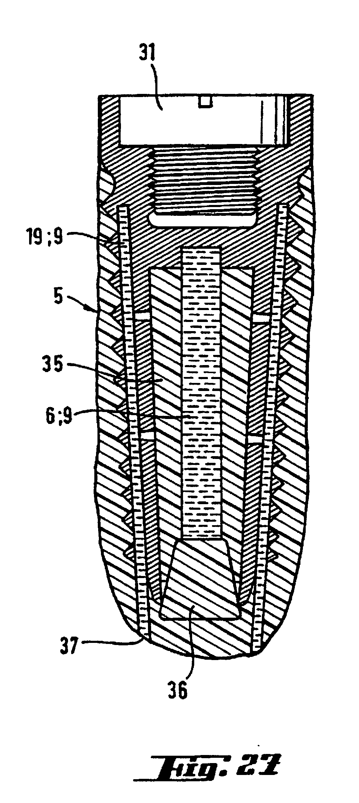

Fig. 26: Nachdem die Gusshöhlung 2 in der Formmasse 1 mit dem bloabbaubaren Material gefüllt worden ist, wird der Hohlkörper 18 in die Gusshöhlung 2 eingesetzt, wobei alle Formkeme 23,24 mit ihren Spitzen 33,38 In die Formmasse hineingestochen werden. Nach dem Abbinden bzw. Aushärten des bioabbaubaren Materials werden die Formkeme 23,24 unten aus dem Implantat 5 herausgezogen und die vorhandenen Räume, also Reservoire, mit entsprechenden medizinischen Substanzen gefüllt. Danach wird der Innenraum 15 unten durch den zapfenförmigen Verschluss 36 verschlossen und die längs der Aussenwand des Implantat-Kerns verlaufenden Räume 19 bei den unteren Oeffnungen 37 mittels Reibungswärme zusammengeschweisst, so dass das Implantat nach Figur 27 erhalten wird.

Fig. 27: Fertiggestelltes Implantat mit einem Implantat-Kem nach Figur 25.

In einer weiteren Variante, welche hier nicht durch Zeichnungen dargestellt ist, bringt man einen Formkem in die Gusshöhlung 2 ein, welcher senkrecht von der oberen Oberfläche bis zum tiefsten Punkt der Gusshöhlung 2 verläuft. Dieser Formkem ist in der Formmasse 1 so angeordnet, dass man nach dem Einfüllen des plastischen Materials in die Gusshöhlung (2) und deren Erhärten sowie Entfernen des gebildeten Implantates aus der Formmasse und Entfernen des Formkems aus dem Implantat an der Stelle des Formkerns ein gegebenenfalls mit einem Gewinde versehener Stift (Fixationsstift bzw. -schraube) aus einem biokompatiblen, aber nicht osseointegrierbaren Material, z.B.Edelstahl, eingesetzt ist. Dessen Durchmesser am Schaft ist kleiner als der Durchmesser des Formkems. Dabei bildet sich ein Zwischenraum, den man mit bioaktiver Substanz füllen kann. in einer weiteren Anordnung kann der Schaft des mit einem Gewinde versehenen Stiftes länger als der Formkern sein und den tiefsten Punkt der Gusshöhlung (2) mit seinem Gewinde durchschneiden. Die so verlängerte, ihr Gewinde selbst schneidende Fixationsschraube wird über den tiefsten Punkt der Extraktionsalveole in den Kieferknochen eingedreht.

Wird in der Heilungsphase das bioabbaure Material unter Einwirkung des biologisch aktiven Materials durch Knochenmaterial ersetzt, ist die Fixationsschraube allseitig von Knochenmaterial umgeben. Da diese Schraube nicht osseointegrierbar ist, kann sie leicht entfernt werden. Der zurückbleibende Kanal im Kieferknochen ermöglicht das Einsetzen einer exakt angepassten Fixtur in natürlicher, achsengerechter Richtung. Alternativ hierzu kann man eine geeignet geformte osseointegrierbare Fixationsschraube im Kieferknochen belassen und diese als Fixtur mit einer Implantatsuprastruktur versehen.

Der Vorteil dieser Varianten besteht darin, dass mit einer therapeutischen Massnahme eine Extraktion und eine Implantation erfolgen kann. Durch die Anwesenheit biologisch aktiver Substanzen an der Oberfläche des Implantats findet ein besonders wirksamer Wundverschluss des angrenzenden Gewebes statt sowie eine schnelle Osseointegration der Implantatfixtur.

## Patentansprüche

1. Medizinisches Implantat (5) zum Einsetzen in einen Raum mit einem vorgegebenen Ausmass, **erhältlich durch:**
Ausfüllen einer Cusshöhlung (2) mit einem sich abbindenden plastischen Material mit mineralischen Bestandteilen auf der Basis von Calcium und Phosphat oder einem bei der Knochenneubildung bioabbaubaren Material oder Kombinationen davon; **gekennzeichnet durch**:
Anordnen mindestens eines Formkerns (3; 23; 24) in der Gusshöhlung (2) vor dem Einfüllen des abbindbaren Materials;
Entnehmen des gebildeten Implantats (5) mit dem mindestens einen Formkern nach dem Abbinden des plastischen Materials aus der Gusshöhlung (2);
Entfernen des wenigstens einen Formkernes aus dem Implantat, wobei ein vom Innenbereich des Implantats (5) bis zu dessen Aussenfläche verlaufender Raum (6; 19) als Reservoir gebildet wird, das mit einer biologischen Substanz aufgefüllt wird.

2. Medizinisches Implantat (5) nach Anspruch 1, erhältlich durch Formen der Gusshöhlung (2) als Abdruck eines extrahierten Zahnes oder eines Knochenresektates.

3. Medizinisches Implantat (5) nach Anspruch 1 oder 2, erhältlich durch Anordnen von drahtförmigen oder stäbchenförmigen Formkernen (3) in der Gusshöhlung (2), welche nach ihrer Entnahme aus dem Implantat Reservoire in Form von kanal- oder kapillarförmigen Räumen bilden.

4. Medizinisches Implantat (5) nach einem der vorangehenden Ansprüche, erhältlich durch Verwenden von osteokondukiven Polymeren als bioabbaubarem Material.

5. Medizinisches Implantat (5) nach einem der vorangehenden Ansprüche, erhältlich durch Verwenden eines Wirkstoffs mit osteokonduktiven Eigenschaften als biologisch aktive Substanz.

6. Medizinisches Implantat (5) nach einem der vorangehenden Ansprüche, erhältlich durch Verwenden eines Proteins mit den Eigenschaften eines transformierenden Wachstumsfaktors vom Typ Beta (TGF-β) oder Kombinationen davon als biologisch aktiver Substanz.

7. Verfahren zur Herstellung eines medizinischen Implantates (5) durch Bilden einer Gusshöhlung (2) in einer Formmasse (1) in Form eines Abdruckes eines extrahierten Zahnes oder eines Knochenresektates und Einfüllen einer sich abbindenden Masse mit mineralischen Bestandteilen auf der Basis von Calcium und Phosphat oder eines bei der Knochenneubildung bioabbaubaren Materials oder Kombinationen davon in diese Gusshöhlung (2), **dadurch gekennzeichnet, dass** vor dem Einfüllen der sich abbindenden Masse in der Gusshöhlung (2) mindestens ein Formkern (3; 23; 24) angeordnet wird, dass dieser nach dem Einfüllen und Abbinden des plastischen Materials zusammen mit dem Implantat aus der Gusshöhlung (2) und auschließend aus dem gebildeten Implantat entfernt wird, und daß nach seiner Entfernung aus dem Implantat ein von Innenbereich des Implantats (5) bis zu dessen Aussenfläche verlaufender Raum (6; 19) als Reservoir gebildet wird, das mit einer biologisch aktiven Substanz aufgefüllt wird.

8. Verfahren gemäss Anspruch 7, **dadurch gekennzeichnet, dass** man in die Gusshöhlung (2) eine alloplastische, osseointegrierbare Fixtur (18) mit Formkernen (23, 23A) einsetzt.

9. Verfahren gemäss Anspruch 7, **dadurch gekennzeichnet, dass** man ein medizinisches Implantat als Abgabesystem für biologisch wirksame Stoffe herstellt.

## Claims

1. Medical implant (5) for insertion into a space with a given dimension, **obtainable by:**
filling a cast cavity (2) with a setting plastic material with mineral constituents based on calcium and phosphate or a material which is biodegradable during bone regeneration or combinations thereof; **characterized by**:
arrangement of at least one mandrel (3; 23; 24) in the cast cavity (2) before introduction of the setting material;
removal of the implant (5) formed with the mandrels, of which there are at least one, from the cast cavity (2) after setting off the plastic material;
removal of the mandrel, at least one of which is present, from the implant, whereby a space (6; 19) which runs from the inner region of the implant (5) to its outer surface is formed as a reservoir which is filled with a biological substance.

2. Medical implant (5) according to claim 1, obtainable by shaping the cast cavity (2) as an impression of an extracted tooth or of a bone resection.

3. Medical implant (5) according to claim 1 or 2, obtainable by arranging in the cast cavity (2) filament-shaped or rod-shaped mandrels (3) which, after their removal from the implant, form reservoirs in the form of channel- or capillary-shaped spaces.

4. Medical implant (5) according to one of the preceding claims, obtainable by using osteoconductive polymers as the biodegradable material.

5. Medical implant (5) according to one of the preceding claims, obtainable by using an active compound with osteoconductive properties as the biologically active substance.

6. Medical implant (5) according to one of the preceding claims, obtainable by using a protein with the properties of a transforming growth factor of the beta type (TGF-β) or combinations thereof as the biologically active substance.

7. Process for the production of a medical implant (5) by formation of a cast cavity (2) in a moulding composition (1) in the form of an impression of an extracted tooth or of a bone resection and introduction of a setting composition with mineral constituents based on calcium and phosphate or of a material which is biodegradable during bone regeneration or combinations thereof into this cast cavity (2), **characterized in that,** before the introduction of the setting composition, at least one mandrel (3; 23; 24) is arranged in the cast cavity (2), **in that**, after introduction and setting of the plastic material, this is removed, together with the implant, from the cast cavity (2) and then from the implant formed, and **in that** after its removal from the implant, a space (6; 19) running from the inner region of the implant (5) to the outer surface thereof is formed as a reservoir which is filled with a biologically active substance.

8. Process according to claim 7, **characterized in that** an alloplastic, osteointegratable fixture (18) with mandrels (23, 23 A) is inserted into the cast cavity (2).

9. Process according to claim 7, **characterized in that** a medical implant is produced as a release system for biologically active substances.

## Revendications

1. Implant médical (5) pour une application dans un espace d'une dimension prédéfinie, que l'on peut obtenir par :
remplissage d'une cavité de coulage (2) avec un matériau plastique durcissant avec des composants minéraux à base de calcium et de phosphate ou un matériau biodégradable lors de la régénération osseuse ou une combinaison de ceux-ci, **caractérisé par**
la mise en place d'un moins un noyau de moulage (3; 23; 24) dans la cavité de coulage (2) avant le remplissage avec le matériau durcissable ; le retrait de l'implant (5) formé avec au moins un noyau de moulage hors de la cavité de coulage (2) après la prise du matériau plastique ;
le retrait d'au moins un noyau de moulage de l'implant, un espace (6; 19) s'étendant de la partie interne hors de l'implant (5) jusqu'à sa surface externe constituant un réservoir, qui est rempli d'une substance biologique.

2. Implant médical (5) selon la revendication 1, obtenu par le moulage de la cavité de coulage (2) comme empreinte d'une dent extraite ou d'une résection osseuse.

3. Implant médical (5) selon la revendication 1 ou 2, obtenu par la mise en place de noyaux de moulage (3) en forme de filin ou en forme de bâton dans la cavité de coulage (2), lesquels forment, après leur retrait de l'implant, des réservoirs sous la forme d'espaces ressemblant à un canal ou à un capillaire.

4. Implant médical (5) selon l'une quelconque des revendications précédentes, obtenu par utilisation de polymères ostéoconducteurs comme matériau biodégradable.

5. Implant médical (5) selon l'une quelconque des revendications précédentes, obtenu par utilisation d'une substance active avec des propriétés ostéoconductrices comme substance biologiquement active.

6. Implant médical (5) selon l'une quelconque des revendications précédentes, obtenu par utilisation d'une protéine avec les propriétés d'un facteur de croissance transformant de type bêta (TGF-β) ou des combinaisons de ceux-ci comme substance biologiquement active.

7. Procédé de fabrication d'un implant médical (5) par la formation d'une cavité de coulage (2) dans une matière à mouler (1) sous la forme d'une empreinte d'une dent extraite ou d'une résection osseuse et par le remplissage avec un matériau durcissant avec des composants minéraux à base de calcium et de phosphate ou d'un matériau biodégradable lors de la régénération osseuse ou d'une combinaison de ceux-ci dans cette cavité de coulage (2), **caractérisé en ce qu'**au moins un noyau de moulage (3; 23; 24) est mis en place dans la cavité de coulage (2) avant le remplissage avec la matière durcissante, **en ce que** celui-ci est retiré de la cavité de coulage (2) après remplissage et prise du matériau plastique conjointement avec l'implant et finalement de l'implant formé et **en ce que**, après son retrait de l'implant, un espace (6, 19) s'étendant de la partie interne de l'implant (5) jusqu'à sa surface externe se forme comme réservoir, qui est rempli avec une substance biologiquement active.

8. Procédé selon la revendication 7, **caractérisé en ce qu'**on insère dans la cavité de coulage (2) une fixation alloplastique ostéointégrée (18) avec des noyaux de moulage (23; 23A).

9. Procédé selon la revendication 7, **caractérisé en ce qu'**on fabrique un implant médical comme système de distribution de substances biologiquement actives.
